# EUROPEAN PATENT APPLICATION

(11) **EP 2 042 075 A1**
(43) Date of publication of application: **01.04.2009**
(21) Application number: 08015308.3
(22) Date of filing: 29.08.2008
(51) Int. Cl.: A61B 1/00, A61B 1/04

(54) **Electronic endoscope**

(30) Priority: 26.09.2007 JP 2007248401
(71) Applicant: Fujinon Corporation, Saitama-shi, Saitama (JP)
(72) Inventor: Ohki, Toshio, Saitama (JP)
(74) Representative: Klunker . Schmitt-Nilson . Hirsch

(57) **Abstract**

An electronic endoscope apparatus comprises a processor unit so as to allow an A type scope not having a timing generator and a B type scope having a timing generator to be connector-connected to the processor unit. The electronic endoscope comprises: an in-client-circuit timing generator and a first image signal processing unit both corresponding the A type scope; and a second image signal processing unit corresponding to the B type scope, wherein control is provided such that when the A type scope is connected to the processor unit, the in-client-circuit timing generator and the first image signal processing unit are operated, whereas when the B type scope is connected to the processor unit, the second image signal processing unit is operated.

## Description

### Background of the Invention

### 1. Field of the Invention

The present invention relates to an electronic endoscope, and more particularly to the configuration of an electronic endoscope apparatus in which a scope not equipped with a timing generator and a scope equipped with a timing generator can be connected to a processor unit which is an image processing apparatus.

### 2. Description of the Related Art

An electronic endoscope apparatus is able to display on a monitor an image of an object to be observed as, for example, the object illuminated with light is imaged by a charge coupled device (CCD) which is a solid-state imaging device mounted in- a scope (electronic endoscope), and an imaging signal from this CCD is supplied to a processor unit (main unit) and is subjected to predetermined signal processing in an image processing circuit within this processor unit. This type of electronic endoscope apparatus is so configured that a plurality of scopes in which types of the aforementioned CCDs and imaging methods (signal processing methods) are different can be connected to the processor unit.

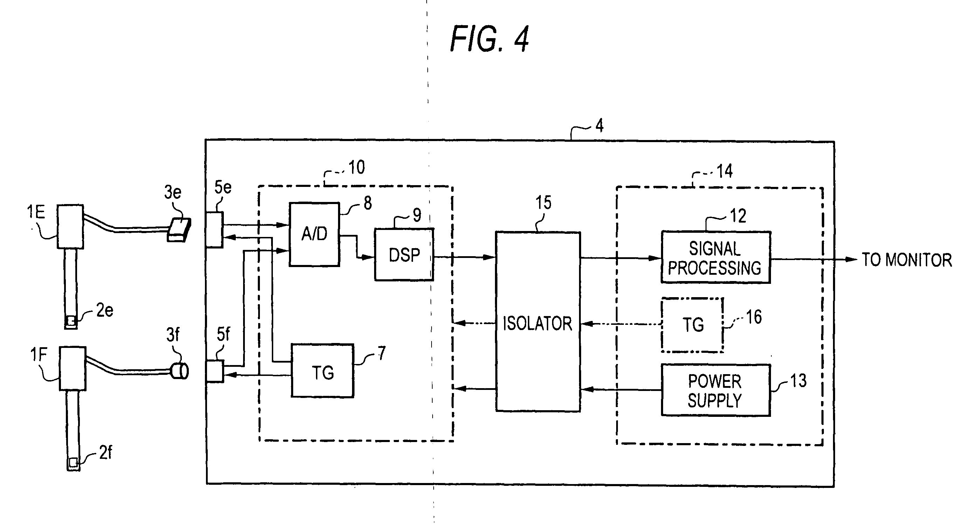

Fig. 4 shows a configuration of the related-art electronic endoscope apparatus. As shown in Fig. 4, for example, an E type scope 1E has a CCD 2e mounted at its leading end portion, and its square-type electric connector 3e is connected to a square type connector receiver 5e of a processor unit 4. Meanwhile, an F type scope 1F has a CCD 2f mounted at its leading end portion, and its round-type electric connector 3f is connected to a round type connector receiver 5f of a processor unit 4. These electric connectors 3e and 3f are for connecting to the processor unit 4 signal lines and the like for transmitting video signals obtained by the CCDs 2e and 2f. Although not shown, lightquides for supplying light source light into the scopes 1E and 1F as illumination light are connected to the processor unit 4 and the like by optical connectors.

In addition, a patient circuit 10 including a timing generator 7, an A/D converter 8, a digital signal processor (DGP) 9, and the like, as well as a secondary circuit 14 including a signal processing circuit 12, a power supply circuit 13, and the like, are provided in the processor unit 4. An electrical isolator 15 constituted by a pulse transformer or a photocoupler is interposed between the patient circuit 10 and the secondary circuit 14.

According to such an electronic endoscope apparatus, by changing the shapes of the electric connectors 3e and 3f and the connector receivers 5e and 5f between the E type and the F type, it is possible to perform image processing and the like which are adapted to the respective types of scopes 1E and 1F while eliminating erroneous connection of the connectors. In addition, electrical safety of the client circuit 10 is ensured as the client circuit 10 is electrically separated by the isolator 15 from the secondary circuit 14 where the power supply circuit 13 which is directly connected to commercial supply is disposed.

In the related-art electronic endoscope apparatus, it is practiced to dispose a timing generator 16 in the secondary circuit 14 shown in Fig. 4, for example (e.g., the aforementioned JP-A-11-289530). In this case, a CCD drive signal (such as a clock signal) outputted from the timing generator 16 is supplied to the CCDs 2e and 2f of the scopes 1E and 1F via the isolator 15 and the client circuit 10. The other timing signals are also supplied to the client circuit 10 via the isolator 15. However, in the case where the CCD drive signal is supplied to the CCDs 2e and 2f via the i9solator 15, there is a problem in that a phase shift and the like can occur in the CCD drive signal and deteriorates the image quality. The higher the pixilation of the CCDs 2e and 2f is and the higher the speed of the CCD drive signal (clock signal) is, the greater the effect of this problem becomes.

Accordingly, in the case of Fig. 4, the arrangement provided is such that the timing generator 7 is disposed in the client circuit 10, and the CCDs 2e and 2f are driven by the CCD drive signal (such as clock signal) from this timing generator 7 to thereby make it possible to obtain an image of the object to be observed with excellent image quality. In addition, to cope with even higher pixilation in recent years, it is also practiced to mount a timing generator into the scope, as shown in the JP-A-2003-93341.

In the related art, however, there are cases where the timing generator 7 for outputting the CCD drive signal is not mounted in the scope (1E, 1F, etc.) and cases where it is mounted therein, so that the processor unit (4) must be fabricated in conformity to the configuration of such scopes. Accordingly, in this case, there has been a problem in that different types of scopes cannot be connected to a single processor unit, entailing higher cost.

### Summary of the Invention

The present invention has been devised in view of the above-described problems, and its object is to provide an electronic endoscope apparatus in which both the scope equipped with the timing generator and the scope not equipped therewith can be used by being connected to a single processor unit, and which makes it possible to form an excellent image by high-speed drive signals corresponding to highly pixilated imaging devices.

To attain the above object, in accordance with a first aspect of the invention there is provided an electronic endoscope apparatus comprising a processor unit so as to allow an A type scope not having a timing generator and a B type scope having a timing generator to be connector-connected to the processor unit, the apparatus comprising: an in-client-circuit timing generator and a first image signal processing unit both corresponding the A type scope; and a second image signal processing unit corresponding to the B type scope, wherein control is provided such that when the A type scope is connected to the processor unit, the in-client-circuit timing generator and the first image signal processing unit are operated, whereas when the B type scope is connected to the processor unit, the second image signal processing unit is operated.

In accordance with a second aspect of the invention, the processor unit further comprises: a D₁ type digital signal processor; and a D₂ type digital signal processor, each of the D₁ type digital signal processor and the D₂ type digital signal processor being connectable to both of the first image signal processing unit and the second image signal processing unit, and either one of the D₁ type digital signal processor and the D₂ type digital signal processor is selectively operated in correspondence with a difference in an image processing type of the scope. It should be noted that the aforementioned A, B, D₁, and D₂ are for distinguishing the types.

According to the configuration of the above-described first aspect of the invention, the type of scope is determined as the processor unit effects communication with the scopes connected thereto. When the A type scope is connected, the timing generator and the first image signal processing unit in the client circuit of the processor unit are turned on. As a CCD drive signal is supplied from this timing generator to the charge coupled device (CCD) of the A type scope, an imaging signal is outputted from the CCD. This imaging signal is supplied to the first image signal processing unit, where the imaging signal is subjected to predetermined signal processing, thereby forming an image of the object to the observed.

Meanwhile, when the B type scope is connected, the second image signal processing unit in the processor unit is turned on. In this case, as a CCD is driven by a CCD drive signal from a timing generator within the scope, an imaging signal is outputted from the CCD. This imaging signal is supplied to the second image signal processing unit, where the imaging signal is subjected to predetermined signal processing, thereby forming an image of the object to the observed.

According to the configuration of the above-described second aspect of the invention, either one of the D₁ type digital signal processor and the D₂ type digital signal processor is selected in correspondence with a difference in the image processing type of the connected scope irrespective of the A type and the B type. Even in cases where there is the difference in the image processing type of scopes of the same type, it is possible to perform excellent image processing. Namely, among the CCDs, there types such as a primary color system (R, G, B) CCD and a complementary color system (Y, Mg, Cy, G) CCD, and there are also differences in the pixel clock signal (or the number of pixels) and the like. The D₁ type digital signal processor or the D₂ type digital signal processor is selected in correspondence with the image processing type of each of such various CCDs.

### Brief Description of the Drawings

Fig. 1 is a circuit block diagram illustrating the configuration of a processor unit (main unit) of an electronic endoscope apparatus in accordance with an embodiment of the invention;
Figs. 2A and 2B are circuit block diagrams illustrating configurations of an A type scope and a B type scope, respectively;
Fig. 3 is a flowchart illustrating the operation of the electronic endoscope apparatus in accordance with the embodiment; and
Fig. 4 is a diagram illustrating a configuration of the related-art electronic endoscope apparatus in which two types of scopes are connected.

### Detailed Description of the Invention

In addition, according to the second aspect of the invention, it is possible to use a digital signal processor corresponding to the difference in the image processing type of the scope irrespective of whether not the scope is a type equipped with a timing generator, and excellent image processing is made possible even if there is the difference between the primary color system the complementary color system, a difference in the number of pixels, and so forth in the imaging devices.

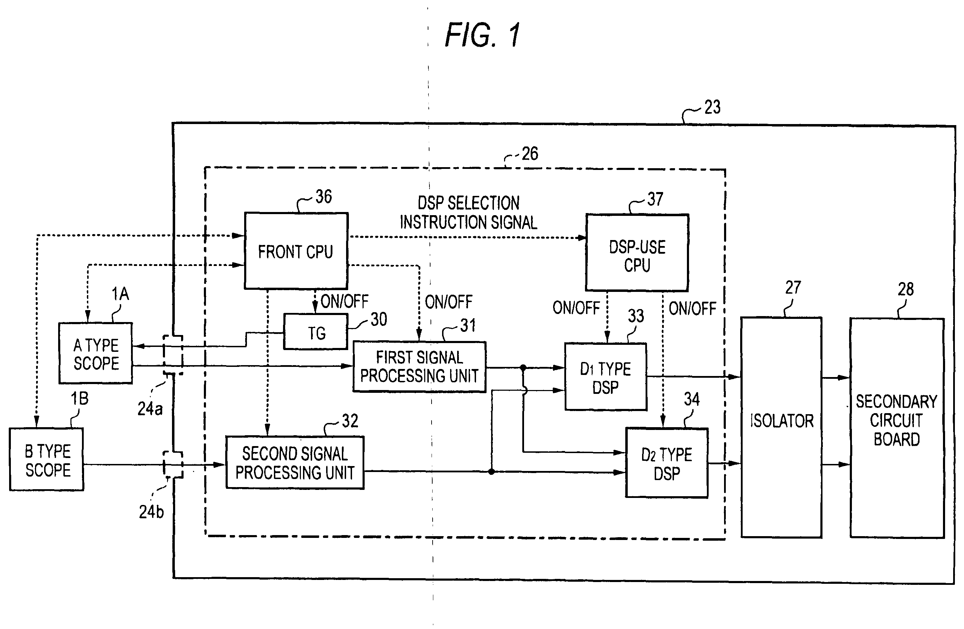

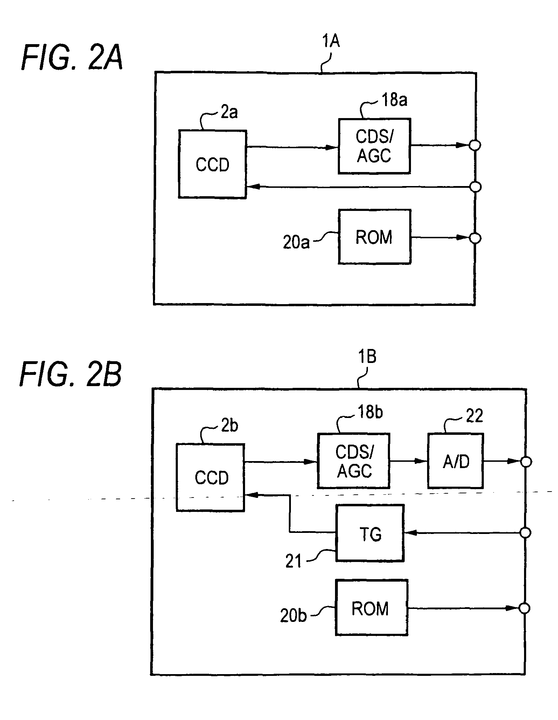

Figs. 1, 2A, and 2B show the configuration of an electronic endoscope apparatus in accordance with an embodiment of the invention. Fig. 1 shows the configuration of a processor unit (main unit), and Figs. 2A and 2B show configurations of A type and B type scopes (electronic endoscopes), respectively. First, as shown in Fig. 2A, an A type scope 1A is not equipped with a timing generator and has a CCD 2a, a correlated double sampling (CDS) / automatic gain control (AGC) circuit 18a, and a read-only memory (ROM) or a microcomputer or the like 20a, and a video signal is outputted as an analog signal.

In addition, as shown in Fig. 2B, a B type scope 1B is equipped with a timing generator (TG) 21, has a CCD 2b, a CDS/AGC circuit 18b, an A/D converter 22, and a ROM 20b, and a video signal is outputted as a digital signal. It should be noted that the A type scope 1A and the B type scope 1B are connected to a processor unit 23 by means of connector portions (connector- and processor unit-side connector receivers) 24a and 24b of different shapes so as to discriminate the two scopes 1A and 1B and prevent erroneous connection.

In Fig. 1, in the processor unit 23, one client circuit board 26 is connected to a secondary circuit board 28 via an isolator (pulse transformer, photocoupler, or the like) 27. A timing generator (TG) 30 corresponding to the aforementioned A type scope 1A, a first (image) signal processing unit 31 having an A/D converter and the like and adapted to effect video processing of an output signal from the A type scope 1A, and a second signal processing unit 32 having a buffer circuit and the like and adapted to effect video processing of an output signal from the B type scope 1B are provided on the client circuit board 26. In addition, a D₁ type digital signal processor (DSP) 33 and a D₂ type DSP 34 are respectively connected to the first signal processing unit 31 and the second signal processing unit 32.

In addition, the client circuit board 26 is provided with a front CPU (or a microcomputer) 36 and a DSP-use CPU 37. This front CPU 36 effects communication with the ROMs (or microcomputers) 20a and 20b on the sides of the scopes 1A and 1B to determine the types (presence or absence of the TG, the type of DSP used, etc.) of the scopes 1A and 1B, and turns on and off the power supply of the timing generator 30, the first signal processing unit 31, and the second signal processing unit 32. The DSP-use CPU 37, upon receiving a DSP selection instruction signal from the front CPU 36, turns on and off the first DSP 33 and the second DSP 34. Namely, as for the scopes 1A and 1B, the type of DSP used is determined by the characteristics and type of an imaging device, i.e., by the type of image processing, irrespective of the presence or absence of the TG, and either corresponding DSP (33, 34) is selected.

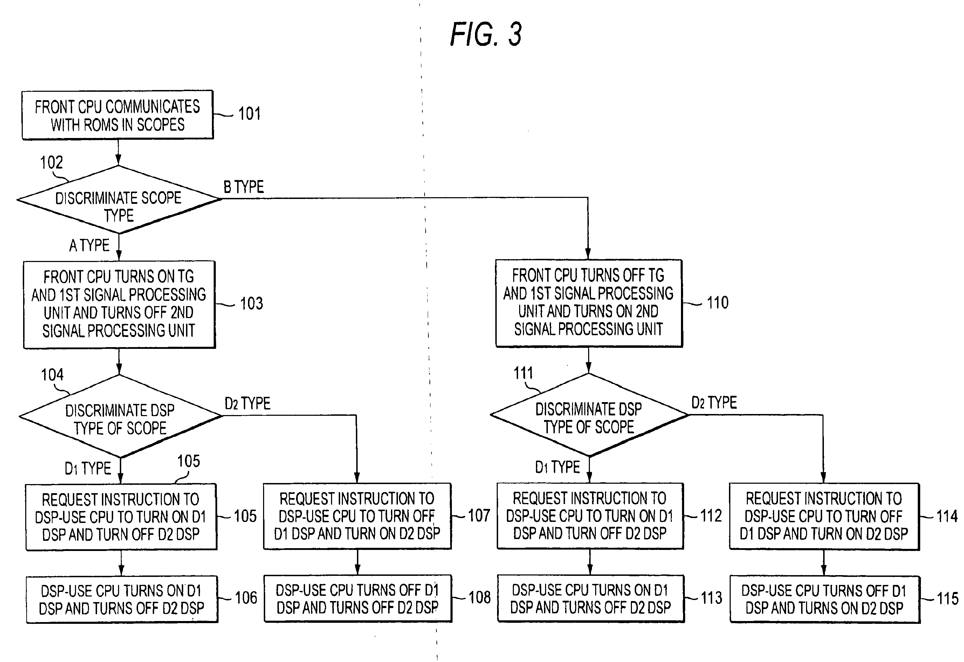

The embodiment has the above-described configuration. As shown in Fig. 1, the electronic endoscope apparatus in accordance with the invention is so configured as to be able to connect the plurality of scopes 1A and 1b to the processor unit 23 by means of the connector portions 24a and 24b having different shapes. When either one of these scopes 1A and 1B is connected, the operation shown in Fig. 3 is executed by the front CPU 36 and the DSP-use CPU 37.

In Fig. 3, first, the front CPU 36 effects communication with the ROMs 20a and 20b in the scopes 1A and 1B (Step 101). In an ensuing Step 102, the type of the connected scope is discriminated, and if it is determined here that the scope is the A type scope 1A, the operation proceeds to Step 103. In Step 103, the front CPU 36 turns on (the power supply of) the timing generator 30 and the first signal processing unit 31 and turns off (the power supply of) the second signal processing unit 32. Then, in Step 104, the type of the scope DSP is discriminated, and if it is determined here that the type of the scope DSP is the D₁ type, the operation proceeds to Step 105. In Step 105, an instruction signal is outputted to the DSP-use CPU 37 to turn on the D₁ type DSP 33 and turn off the D₂ type DSP 34. As a result, the DSP-use CPU 37 turns on the D₁ type DSP 33 and turns off the D₂ type DSP 34 (Step 106).

If it is determined in the aforementioned Step 104 that the type of the scope DSP is the D₂ type, the operation proceeds to Step 107. In Step 107, an instruction signal is outputted to the DSP-use CPU 37 to turn off the D₁ type DSP 33 and turn on the D₂ type DSP 34. As a result, the DSP-use CPU 37 turns off the D₁ type DSP 33 and turns on the D₂ type DSP 34 (Step 108).

Meanwhile, if it is determined in the aforementioned Step 102 that the connected scope is the B type scope 1B, the operation proceeds to Step 110, in which the front CPU 36 turns off the timing generator 30 and the first signal processing unit 31 and turns on the second signal processing unit 32. Then, in Step 111, the type of the scope DSP is discriminated, and if it is determined here that the type of the scope DSP is the D₁ type, the operation proceeds to Step 112. In Step 112, an instruction signal is outputted to the DSP-use CPU 37 to turn on the D₁ type DSP 33 and turn off the D₂ type DSP 34. As a result, the DSP-use CPU 37 turns on the D₁ type DSP 33 and turns off the D₂ type DSP 34 (Step 113) .

If it is determined in the aforementioned Step 111 that the type of the scope DSP is the D₂ type, the operation proceeds to Step 114. In Step 107, an instruction signal is outputted to the DSP-use CPU 37 to turn off the D₁ type DSP 33 and turn on the D₂ type DSP 34. As a result, the DSP-use CPU 37 turns off the D₁ type DSP 33 and turns on the D₂ type DSP 34 (Step 115).

When the A type scope 1A is thus connected, a CCD drive signal is supplied to the CCD 2a by using the timing generator (TG) 30 on the client circuit board 26, whereby an image pickup signal is outputted from the CCD 2a. This image pickup signal is processed by the CDS/AGC circuit 18a, and an analog video signal is outputted from the scope 1A to the processor unit 23. Then, this analog video signal is converted to a digital video signal by the first signal processing unit 31, and this digital video signal is processed by either the D₁ type DSP 33 or the D₂ type DSP 34. An output of this DSP 33 or 34 is outputted to a monitor through signal processing in the secondary circuit board 28, thereby displaying on the monitor an image (video image) of the object to be observed.

Meanwhile, when the B type scope 1B is connected, a CCD drive signal is supplied to the CCD 2b by using the timing generator (TG) 21 on the in the scope 1B, whereby an image pickup signal is outputted from the CCD 2b. This image pickup signal is processed by the CDS/AGC circuit 18b and the A/D converter 22, and a digital video signal is outputted from the scope 1B to the processor unit 23. Then, this digital video signal is subjected to buffer processing by the second signal processing unit 32, and this digital video signal is processed by either the D₁ type DSP 33 or the D₂ type DSP 34, thereby displaying on the monitor an image of the object to be observed through the processing by the secondary circuit board 28.

Although in the above-described embodiment both the front CPU 36 and the DSP-use CPU 37 were used, control of the turning on and off of the power supply of the DSPs 33 and 34 may be effected by using only the front CPU 36 without using this DSP-use CPU 37. According to this arrangement, it is possible to attain a cost reduction.

According to the electronic endoscope apparatus in accordance with the invention, advantages are obtained in that both the scope equipped with the timing generator and the scope not equipped therewith can be used by being connected to a single processor unit, that it is possible to form an excellent image by high-speed drive signals corresponding to highly pixilated imaging devices, and that cost reduction also becomes possible.

The entire disclosure of each and every foreign patent application from which the benefit of foreign priority has been claimed in the present application is incorporated herein by reference, as if fully set forth.

## Claims

1. An electronic endoscope apparatus comprising a processor unit so as to allow an A type scope not having a timing generator and a B type scope having a timing generator to be connector-connected to the processor unit, the apparatus comprising:
an in-client-circuit timing generator and a first image signal processing unit both corresponding the A type scope; and
a second image signal processing unit corresponding to the B type scope,
wherein control is provided such that when the A type scope is connected to the processor unit, the in-client-circuit timing generator and the first image signal processing unit are operated, whereas when the B type scope is connected to the processor unit, the second image signal processing unit is operated.

2. The electronic endoscope apparatus according to claim 1,
wherein the processor unit further comprises: a D₁ type digital signal processor; and a D₂ type digital signal processor, each of the D₁ type digital signal processor and the D₂ type digital signal processor being connectable to both of the first image signal processing unit and the second image signal processing unit, and
either one of the D₁ type digital signal processor and the D₂ type digital signal processor is selectively operated in correspondence with a difference in an image processing type of the scope.
